Europäisches Patentamt

European Patent Office

Office européen des brevets

(11) Publication number: 0 241 139
A2

(12) **EUROPEAN PATENT APPLICATION**

(21) Application number: 87302011.9

(22) Date of filing: 09.03.87

(51) Int. Cl.4: **C12N 15/00** , C12P 21/00 , A61K 39/395

The applicant has filed a statement in accordance with Rule 28 (4) EPC (issue of a sample only to an expert). Accession number(s) of the deposit(s): ATCC No. HB-9017, ATCC No. HB-9016.

(30) Priority: 13.03.86 US 839409

(43) Date of publication of application:
14.10.87 Bulletin 87/42

(84) Designated Contracting States:
AT BE CH DE ES FR GB IT LI LU NL

(71) Applicant: MERCK & CO. INC.
126, East Lincoln Avenue P.O. Box 2000
Rahway New Jersey 07065-0900(US)

(72) Inventor: Bhogal, Balbir S.
704 Murray Street
Avenel New Jersey 07001(US)
Inventor: Gammon, Maureen
197 Charlton Avenue
South Orange New Jersey 07079(US)
Inventor: Murray, Keith P.
104 Conover Lane
Red Bank New Jersey 07701(US)
Inventor: Crane, Mark S.
137 St. Paul's Street
Westfield New Jersey 07090(US)
Inventor: Zemcik, Barbara
10 Alvin Terrace
Springfield New Jersey 07081(US)
Inventor: MacDonald, Thomas T.
37 Hervey Close
Finchley London N3(GB)

(74) Representative: Hesketh, Alan, Dr.
European Patent Department Merck & Co.,
Inc. Terlings Park Eastwick Road
Harlow Essex, CM20 2QR(GB)

(54) Anti-idiotypic vaccine for coccidiosis.

(57) Mouse hybridomas are developed which secrete monoclonal antibody specifically reactive with surface immunogens of Eimeria sporozoites. The monoclonal antibodies are used as immunogens in both rabbits and chickens to produce anti-idiotypic antibodies. The anti-idiotypic antibody is specific for the antigen-binding-site-associated idiotype of the monoclonal antibody, has the structural mirror image of the monoclonal antibody binding site, and is able to function as a surrogate immunogen for Eimeria. Immunization of young chickens with the anti-idiotypic antibody induces a protective immune response against a subsequent Eimeria challenge infection. The anti-idiotypic vaccine produces anti-Eimeria sporozoite antibodies, along with a reduction in cecal lesions and oocyst production following challenge with viable Eimeria sporulated oocysts.

## ANTI-IDIOTYPIC VACCINE FOR COCCIDIOSIS

### BACKGROUND OF THE INVENTION

Coccidiosis is a disease caused by infection with one or more of the many species of coccidia, a subdivision of the phylum protozoa. The coccidia are intracellular parasites which can infect a wide range of hosts including domestic fowl. Coccidial infections in poultry may be either acute and result in devastating flock mortality or chronic and be characterized by lack of weight gain. In either situation the disease results in economically devastating losses to the poultry industry.

Chickens recovering from low grade infections with any of the Eimeria species (spp.) exhibit a protective immunity to reinfection suggesting the control of coccidiosis by the use of vaccines. Indeed, young broiler chickens vaccinated with an extract of Eimeria tenella sporozoites were protected from challenge with virulent E. tenella sporulated oocysts. Furthermore, chickens vaccinated with immunogens isolated from E. acervulina sporozoites were protected against challenge by virulent E. tenella, E. acervulina and E. maxima. The use of vaccines prepared from either the whole parasites or from parasite polypeptides is limited because of the extreme difficulty of growing the parasite in economically sufficient quantities for vaccine production.

The present invention relates to an alternative coccidiosis vaccine based on anti-idiotypic antibodies acting as surrogates for specific Eimeria immunogens. The development of hybridoma technology provides a tool for the production of large quantities of anti-idiotypic antibodies which can be used as a vaccine to protect chickens against coccidiosis. The site on the antibody molecule that makes contact, or binds with the antigen is called the antigen-binding site (paratope). The structural configuration of a paratope includes both the heavy and light chain peptides of the variable region. An idiotope is defined as an antigenic determinant present on the variable (V) region of an antibody molecule. The idiotype of an antibody reflects its unique composition of idiotopes. Idiotypic determinants may or may not be part of the antigen binding site (paratope), however, if they occur in the antigen binding site they are called paratope associated. Conversely, an idiotype located on the non-antigen-binding site of the variable region is termed a non-paratope-associated idiotype. Anti-idiotypic antibodies (Ab-2) are generally derived from monoclonal antibodies (Ab-I) which are immunologically specific for the immunogen which induced their formation. In a situation where an idiotype is paratope associated, the monoclonal antibody (Ab-I), bearing the idiotype expresses a complementary three dimensional structure (paratope). Anti-idiotypic antibody (Ab-2) produced against Ab-I could have the identical three dimensional structure of the antigen even though the anti-idiotype and the antigen may be chemically different. When the immunogen used for the induction of monoclonal antibody is reacted with the antibody in an in vitro serological reaction it is termed an antigen. Mouse derived monoclonal antibodies (Ab-I) are immunogenic by virtue of the pressure of idiotype on their variable regions and thus are capable of inducing antibody production in other species and also in the same species when the animal has a different idiotype. When the idiotope on Ab-I is paratope associated the resulting antibody (anti-Id, Ab-2) bears the internal image of the antigen. The anti-Id or Ab-2 contains a paratope which binds the initial antibody or Ab-I at its idiotope. When the idiotope on Ab-2 is injected into the target animal as a vaccine, it induces an immune response. If the idiotope on Ab-2 is paratope associated then Ab-I and anti-anti-Id will be the internal image of each other and both will bind the paratope of Ab-2. Consequently, Ab-3 will have the capacity to bind the antigen involved in the production of Ab-I because of the mirror image nature of Ab-I and Ab-3.

### OBJECTS OF THE INVENTION

It is, accordingly, an object of the present invention to provide an anti-idiotypic antibody that will induce protective immunity against coccidiosis. Another object is to provide a means for obtaining this anti-idiotypic antibody vaccine. A further object is to produce a hybridoma capable of secreting monoclonal anti-Eimeria sporozoite antibodies. Another object is to provide a composition for the prophylactic administration of this vaccine. A further object is to provide a coccidiosis vaccine which is protective against those forms of Eimeria mainly responsible for economic loss. These and other objects of the present invention will be apparent from the following description.

## SUMMARY OF THE INVENTION

Mouse hybridomas are developed which secrete monoclonal antibody specifically reactive with surface immunogens of Eimeria sporozoites. The monoclonal antibodies are used as immunogens in both rabbits and chickens to produce anti-idiotypic antibodies. The anti-idiotypic antibody is specific for the paratope of the monoclonal antibody and has the structural image of the parasite antigenic structure recognized by the monoclonal antibody binding site. The anti-idiotypic antibody is, thus, able to function as a surrogate immunogen for Eimeria. Immunization of young chickens with the anti-idiotypic antibody induced a protective immune response against a subsequent Eimeria challenge infection. The anti-idiotypic vaccine produces anti-Eimeria sporozoite antibodies and reduces cecal lesions and oocyst production following challenge with viable Eimeria sporulated oocysts.

## DETAILED DESCRIPTION OF THE INVENTION

The present invention relates to the production of hybridomas which secrete monoclonal antibody (mAb) specifically reactive with antigens associated with the sporozoite stage of Eimeria. The invention further relates to a process by which the monoclonal antibodies are used to produce anti-idiotypic antibodies which structurally mimic the immunogen present on the surface of Eimeria sporozoites. The invention also relates to the use of the anti-idiotypic antibody as a vaccine effective against coccidiosis.

While the following description and examples illustrate the present invention with respect to E. tenella, it is to be understood that the present invention is applicable to other Eimeria species. Other species include, but are not limited to, E. acervulina, E. maxima, E. burnetti and E. praecox.

Anti-E. tenella sporozoite antibody producing cells are obtained from the spleens of inbred mice, preferably Balb/c which have been immunized with E. tenella sporozoites. The mice are immunized intraperitoneally with about $1 \times 10^4$ to about $1 \times 10^6$, preferably about $2 \times 10^5$ intact sporozoites per 0.5 ml in an equal volume of an acceptable adjuvant. Such acceptable adjuvants include, but are not limited to, Freund's complete, Freund's incomplete, alum-precipitate, water-in-oil emulsion containing Corynebacterium parvum and tRNA. The mice are given intravenous booster immunizations of about $2 \times 10^5$ E. tenella sporozoites on about days 14, 21 and 63 post primary immunization. At approximately day three after the final booster immunization the mice are tested for anti-sporozoite antibody. Spleen cells from two antibody positive mice producing Anti-E. tenella sporozoite antibodies are fused with murine myeloma cells, SP-2/0 by techniques known to the art. Hybridoma cells are selected by growth in hypoxanthine, thymidine and aminopterin supplemented DMEM. Antibody producing hybridomas are cloned using the soft agar technique of MacPherson, Soft Agar Techniques, in Tissue Culture Methods and Applications, Kruse and Paterson, Eds. Academic Press, 1973.

Discrete colonies are transferred into individual wells of culture plates for cultivation. Culture fluids are collected from individual wells and assayed to determine the presence of anti-E. tenella sporozoite antibody and the isotype of the secreted immunoglobulin. Screening of the culture fluid reveals hybridomas producing anti-E. tenella sporozoite antibodies. Since antibodies raised against E. tenella surface antigens protect against infection, all hybridoma monoclonal antibodies are screened for the ability to agglutinate, lyse and specifically bind to the surface of E. tenella sporozoites by standard serological procedures. Two hybridomas producing sporoite surface reactive mAb are designated 1073.10 (1073) and 15-1 with the corresponding mAb designated mAb 1073 and mAb 15-1. Utilizing the Ouchterlony double diffusion technique and goat anti-mouse isotype serum both monoclonal antibodies are of the IgG isotype while mAb 1073 is of the subclass $IgG_3$ and 15-1 is of the subclass $IgG_2$. The two monoclonals react specifically with the surface of E. tenella sporozoites as shown by an immunofluorescense assay.

Both monoclonal antibodies are produced in vivo by injecting pristane primed Balb/C mice, approximately 0.5 ml per mouse, with about $2 \times 10^6$ to about $6 \times 10^6$ hybridoma cells about 4 days after priming. Ascites fluid is collected at approximately 8-12 days and the monoclonal antibodies precipitated with ammonium sulfate, about 35% to about 60% of saturation, with 45% being preferred, washed and resuspended in a physiologically acceptable buffer at a pH of about 7.2. Such physiologically acceptable buffers include, but are not limited to, phosphate buffered saline, phosphate buffered saline glucose, buffered saline and the like.

The anti-E. tenella monoclonal antibodies are further purified by affinity chromatography using a protein A Sepharose matrix and the technique of Ey et al., Immunochemistry 15:429-436 (1978). The purified monoclonal antibodies, are neutralized with about 1M phosphate buffer at about pH 8.0.

In vivo produced anti-E. tenella monoclonal antibodies induce a high degree of passive protection, in one-day-old chickens against E. tenella infection, when about I.5 mg to about 3.5 mg of mAb I073 or about 0.6 mg to about 2.6 mg mAb I5-I is administered at about 24 and about 2 hours prior to sporulated oocysts challenge.

The purified anti-E. tenella monoclonal antibodies are used to induce anti-Id antibodies in mammalian or avian species. Acceptable mammalian species include; but are not limited to, mice, rats, guinea pigs, hamsters, sheep and goats, with rabbits being preferred. New Zealand white rabbits are injected with the purified monoclonal antibodies in adjuvant, preferably Freund's complete adjuvant. Each animal is immunized with about I ml of a 50% adjuvant, 50% antibody mixture containing about 80 $\mu$g to about 300 $\mu$g of either mAb I073 or mAb I5-I at multiple intramuscular (IM) and subcutaneous (SC) sites. The rabbits are boosted at about weekly intervals for three weeks with about 80 $\mu$g of the appropriate monoclonal antibody in Freund's complete adjuvant via SC and IM routes. Two weeks after the last SC-IM immunization the rabbits are boosted with about an equal amount of the appropriate monoclonal antibody in saline by the intravenous route. Approximately 7 days after the final booster immunization the rabbits are bled and the sera collected.

Acceptable avian species include, but are not limited to, pigons, turkeys, guinea fowl and chicks, with chickens being preferred. Chicken anti-Id antibody is prepared by immunizing 6 to 8 week-old Leghorn chickens with about 0.5 mg to about 5 mg of the purified monoclonal antibody in an equal volume of adjuvant, preferably Freund's complete adjuvant via the IM route. The birds are given two weekly booster immunizations of the same monoclonal antibody and at the same concentration. Approximately one week after the final booster the chickens are bled and the sera collected.

The rabbit and chicken anti-Id sera are rendered monospecific by a series of absorptions with Sepharose 4B conjugated normal mouse immunoglobulin (NMIg) and mouse monoclonal antibody which is of the same isotype as mAb I073 and mAb I5-I. The immunoabsorbants are prepared according to the technique of Ey etal., Immunochem. I5: 429-436 (I978). Monospecificity was confirmed by an enzyme-linked immunosorbant assay (ELISA) and competitive inhibition binding of alkaline phosphatase (AP)-conjugated anti-Id to homologous monoclonal antibody by different unlabeled anti-Id preparations. Excess un-conjugated anti-Id I073 induces a I00% inhibition of AP-conjugated anti-Id I073 binding to homologous mAb I073 while excess anti-Id I5-I induces only a 43% inhibition of AP-labeled anti-Id I073 binding to mAb I073. Other anti-Id antibodies raised against monoclonal anti-E. tenella do not compete with anti-Id I073 or anti I5-I for the homologous mAb.

Paratope-associated Id determinants are assayed by (a) the ability of anti-Id to inhibit the binding of homologous monoclonal antibody to sporozoite antigen immobilized on a plastic surface and (b) by the ability of free sporozoite antigen to inhibit the binding of monoclonal antibody to homologous anti-Id antibody. The soluble sporozoite antigen causes only partial inhibition of the binding of anti-Id I073 to its homologous mAb (5I%) and anti-Id I5-I to its homologous mAb (39%). Thus, the Id determinants of both mAb I073 and mAb I5-I are associated with the paratope.

Chickens are immunized by inoculation of anti-Id antibodies, anti-Id I073 or anti-Id I5-I, raised in either rabbits or chickens. Anti-Id antibodies, in a physiologically acceptable medium, are administered either with or without an acceptable adjuvant with alum-precipitate being preferred. Such physiologically acceptable media include, but are not limited to, physiological saline, phosphate buffered saline, phosphate buffered saline glucose, buffered saline and the like. Immunization of chickens starting at about day 2 and continuing at about weekly intervals for about 3 weeks results in immunity to infection. Protective immunity is achieved by administration of from about 25 $\mu$g to about 400 $\mu$g of anti-Id immunogen per chicken on from about I to about 4 separate weekly occasions.

Immunized chickens produce elevated levels of anti-E. tenella sporozoite antibodies following immunization with anti-Id antibodies and after challenge infection.

The following examples illustrate the present without, however, limiting the same thereto.


## EXAMPLE I

### Production of Hybridomas

Anti-E. tenella sporozoite antibody producing cells were obtained from the spleens of Balb/c mice which had previously been immunized with E. tenella sporozoites. A group of mice was immunized intraperitoneally with 2 $\times$ I0$^5$ sporozoites, purified by DE-52 anion exchange chromatography following the method of Schmatz, et al, J. Protozol. 3I:I8I-I93 (I984), per 0.5 ml in an equal volume of Freund's complete adjuvant.

The mice were given intravenous booster immunizations containing $2 \times 10^5$ purified E. tenella sporozoites in phosphate buffer on days 14, 21 and 63 following the initial immunization. The mice were tested for anti-E. tenella sporozoite antibody beginning on the third day after the final booster immunization. Spleens were collected from anti-sporozoite antibody producing mice and single cell suspensions prepared in RPMI 1640 culture medium.

Hybridoma production was accomplished by mixing the anti-E. tenella sporozoite antibody producing splenic lymphocytes with hypoxanthine-aminopterin-thymidine (HAT) sensitive murine myeloma cells, SP-2/0, at a cell ratio of 5:1 in Dulbecco's Modified Eagle's Medium (DMEM). Cell fusion was achieved by the addition of 0.3 ml per spleen of a 40% solution of polyethylene glycol, 1000 mol. wt., and gently tapped to resuspend the pellet. The cells were immediately centrifuged at 1,500 RPM for 3 minutes and left for a further 5 minutes before removing the supernatant fluid. The cells were gently resuspended in 10 ml of fresh DMEM. After centrifugation at 1,000 rpm for 7 minutes the supernatant fluid was removed, the cells were resuspended in 60 ml of fresh DMEM supplemented with 10% fetal calf serum, $10^{-4}$M hypoxanthine, $10^{-5}$M thymidine and plated in 24-well culture dishes at a density of $2.5 \times 10^5$ spleen cells per 0.5 ml per well. After overnight incubation at 37°C the culture media was replaced with DMEM containing 10% fetal calf serum, $10^{-4}$M hypoxanthine, $10^{-5}$M thymidine and $8 \times 10^{-7}$M aminopterin. Hybridoma selection in the HAT medium was terminated after 7 days and the cultures were maintained in DMEM until anti-sporozoite antibodies were detected in the culture fluid using a solid phase immunoradio assay (SPIRA). Polyvinyl plates (96 well) were coated with E. tenella sporozoite antigen. The antigen was prepared by sonicating DE-52 purified E. tenella sporozoites and diluting the supernatant fluid with 0.05M carbonate buffer, pH 9.6, to a protein concentration of 25 $\mu$g/ml. One hundred microliters of the antigen was added to each well, 100 $\mu$g/well, and incubated at 37°C for 4 hours. The wells were washed 3 times with phosphate buffered saline (PBS) supplemented with 1.5% horse serum (HS). The wells were blocked with 200 $\mu$l PBS containing 10% HS at 37°C for 4 hours. The culture fluids from the hybridoma cultures were diluted, 100 $\mu$l of each appropriate dilution in PBS/1.5% HS was added to each well and incubated at 37°C for 2 hours. The wells were washed and $^{125}$I-goat anti-mouse serum, 100 $\mu$l diluted in PBS/1.5% HS, was added and the plates incubated for 2 hours. The plates were washed, the wells removed and counted. Antibody producing cells from the individual wells were cloned by dilution and culture in soft agar using the technique of MacPherson, Soft Agar Techniques, in Tissue Culture Methods and Applications, Kruse and Paterson, Eds. Academic Press, (1973). The antibody producing hybridoma cells were serially diluted to obtain 10 to 30 cells per well and added to 0.2% agar in DMEM containing 10% fetal calf serum. After incubation for 10 days at 37°C in an atmosphere of 5% $CO_2$ 95% air, 12 discrete colonies are selected and transferred to one each of 96 wells containing 200 $\mu$l DMEM and 10% fetal calf serum. The clones were cultured for 7 days, after which the culture fluids are collected and assayed in the SPIRA test. Two of these clones were designated 1073.10 (1073) and 15-1 and were used for further study.

A sample of hybridoma 1073.10 was deposited in the American Type Culture Collection, 12301 Parklawn Drive, Rockville, Maryland 20852, USA, on February 11, 1986 and has been assigned ATCC number HB9017. A sample of hybridoma 15-1 was also deposited in the American Type Culture Collection on February 11, 1986 and has been assigned ATCC number HB9016.

The isotype of the monoclonal antibodies was determined by the Ouchterlony double diffusion test employing goat anti-mouse isotype serum. The mAb secreted by hybridoma 1073 exhibited an IgG3 isotype while the antibody from the 15-1 hybridoma had an IgG2 isotype.

EXAMPLE 2

Production and Purification of Monoclonal Antibodies

Hybridoma cells producing mAb 1073 and mAb 15-1 from Example 1 were grown in DMEM supplemented with 10% fetal calf serum to a cell density of approximately $1 \times 10^6$ cells per ml. The cells were collected, washed three times with DMEM and resuspended in DMEM at a concentration of $4 \times 10^6$ cells per ml. Balb/c mice were primed with pristane, 0.5 ml, intraperitoneally 4 days prior to the intraperitoneal injection of $2 \times 10^6$ hybridoma cells per animal. Ascites fluid was collected from the peritoneal cavity between 8 and 12 days after the hybridoma injection. The ascites fluid was centrifuged to remove any cells and the immunoglobulin (Ig) precipitated. The ascites fluid was combined with saturated ammonium sulfate to reach a 45% saturation. The precipitate was allowed to stand at 0°C for 2 hours and then resolublized in 0.1M borate buffer, pH 8.2. The solution was dialyzed against the resolubilization buffer plus azide (0.02%) and phenylmethanesulfonic acid (PMSF) (0.0001N) (Buffer I) and further purified by protein A affinity chromatography using the technique of Ey et al., Immunochem. 15:429-436 (1978). The protein-A affinity matrix had a binding capacity of 20 mg IgG/Cm³ gel The column was equilibrated with Buffer I and the mAb sample applied. The column was washed with Buffer I until the effluent was free of 280 nm absorbing material. The mAb IgG was eluted with 0.2M glycine-HCl buffer, pH 3.0. The fractions containing IgG were pooled and neutralized with 1/10 volume of IM phosphate buffer, pH 8.0. The purified mAb was dialyzed against phosphate buffer and stored.

The purified mAb 1073 and mAb 15-I were assayed for antigen binding specificity by both an indirect immunofluorescense and an enzyme-linked immunoassay. Antibody binding to intact sporozoites was determined by reacting $5 \times 10^5$ sporozoites in a volume of 100 μl PBS/azide containing 1% bovine serum albumin (BSA) with 5 μg of either of the monoclonal antibodies and incubated at 4°C for 30 minutes. The sporozoite-mAb complex was washed and fluorescein-conjugated rabbit anti-mouse Ig was added and incubated at 4°C for 30 minutes. The sporozoites were washed, resuspended in 0.5 ml PBS/azide and analyzed in a Fluorescence Activated Cell Sorter. Both mAb 1073 and mAb 15-I reacted specifically with the surface of intact sporozoites. The ability of the monoclonal antibodies to bind sporozoite antigen was demonstrated with an enzyme-linked immunosorbant assay (ELISA). Sporozoite antigen was prepared by sonicating sporozoites and diluting the material in 0.05M carbonate buffer, pH 9.6, to a protein concentration of 25 μg per ml. Microtiter wells, 96 per plate, were coated with the sonicated sporozoite antigen, 2.5 μg per well, washed, blocked with bovine serum albumin (BSA) and washed. A 1:1000 dilution of the appropriate mAb was added, washed and a revealing agent added. The revealing agent was alkaline phosphatase (AP) conjugated goat anti-mouse IgG. After incubation for I hour at room temperature the plates were washed and the bound AP activity was detected by the addition of p-nitrophenyl phosphate disodium, I mg/ml. Following a I hour incubation period at 37°C the reaction was read at 405 nm on an automated ELISA reader. Both monoclonal antibodies showed a high degree of reactivity in binding to the soluble sporozoite antigen.

Sporozoite antigen was prepared as detailed in Example I and separated into individual polypeptides according to size by sodium dodecyl sulfate (SDS) polyacrylamide gel electrophoresis (PAGE). Fifty μg of antigen was separated on a 5-20% linear polyacrylamide gradient overlaid with a 3% polyacrylamide stacking gel in the presence of 0.1% SDS and 2-mercaptoethanol, Laemmli, Nature, 227:680-685 (1970). The polypeptides were electrophoresed at 50 ma for 3-4 hours at 10-12°C until the tracking dye, bromophenol blue, was within I cm of the bottom. After separation they were transferred to nitrocellulose paper following the procedures of Erickson et al., J. Imm. Meths. 51:241-249 (1982) and Towbin et al., P.N.A.S. 76:4350 (1979). A Biorad Transblot apparatus was used for this transfer with a voltage gradient of 3.5 V/cm being applied for 21 hours at 11°C.

Each monoclonal antibody, mAb 1073 and mAb 15-I, was reacted with the separated antigens. The monoclonal antibodies were diluted 1:100 with 0.25% gelatin-TEN (0.05 M tris, 0.14 M NaCl, 0.005 M EDTA, 0.05% Triton × 100). After washing, goat anti-rabbit IgG conjugated to horseradish peroxidase was added to the nitrocellulose paper containing the transferred polypeptides. The peroxidase substrate 4 chloro-I-napthol was applied to the nitrocellulose paper and formed a visible reaction product at the site of the polypeptide-antibody complex. No visible reaction was seen in the 1073 blot indicating that under the conditions of the test this antibody does not recognize a sporozoite polypeptide. This finding suggests that 1073 may recognize a non-protein antigen which is found on the surface of the sporozoite. Using the immunoblot procedure mentioned above, monoclonal antibody 15-I is found to react with a E. tenella sporozoite polypeptide of 82KD.

Each monoclonal antibody was able to passively protect chickens against challenge with an infective dose of sporulated oocysts. One day old chickens were injected with either 2.5 mg mAb 1073 or 1.6 mg of mAb 15-I at 24 and 2 hours prior to challenge with E. tenella sporulate oocysts. The lesions were scored on day 6 post challenge. Non-protected control chickens had a mean caecal lesion score of 3.0. Chickens treated with mAb 1073 exhibited a mean caecal lesion score of 1.8 while mAb 15-I produced a lesion score of 1.7, demonstrating a high level of protection against E. tenella infection.

EXAMPLE 3

Production and Characterization of Anti-Id Antibodies in Rabbits

Purified anti-E. tenella sporozoite monoclonal antibodies from Example 2 were used to produce anti-Id 1073 and anti-Id 15-1 antibodies in rabbits. New Zealand White rabbits were immunized by three intramuscular (IM) and subcutaneous (SC) injections of the appropriate mAb in Freund's complete adjuvant. A total of 80 μg of the mAb in 0.5 ml was emulsified with 0.5 ml of Freund's complete adjuvant to give I ml of a water-in-oil emulsion. The injections were given at multiple sites on the legs and back of each rabbit. The animals were boosted at weekly intervals for three weeks with 80 mg of the appropriate mAb in Freund's complete adjuvant by a combination of SC and IM routes. Two weeks after the last IM-SC injection the rabbits were given an intravenous injection of 80 μg of the appropriate mAb in buffered saline. The serum was collected 7 days after the final injection, processed and frozen.

The anti-Id antibodies were purified by a series of absorptions using Sepharose 4B immunoabsorbant with various globulin fractions or monoclonal antibodies acting as the affinity matrix. Preparation of the immunoabsorbants followed the procedure of Ey et al. Immunochem. 15:429-436 (1978). Absorbants included pooled normal mouse gamma globulin (NMIg) and mAb which shared isotype, allotype, and light chain specificity with the specific mAb used to induce the anti-Id. The mAbs used for absorption did not share the ability to bind to E. tenella sporozoite antigen. Normal rabbit serum was prepared in the same manner as a control. The homogeneity of the anti-idiotypic antibodies was confirmed by an ELISA test, see Example 2. Dilutions of each anti-Id antibody (l00 μl) were added to wells containing adsorbed mAb anti-sporozoite antibodies (5.0 μg). The anti-Id antibody was diluted to contain from 0.0l μg to l00,000 μg protein in l ml. Protein concentration was determined by the method of Lowry. The following results were obtained.

## Anti-Idiotypic Antibody

| mAb | Anti-Id Concentration Ng/(ml) | 1073 Absorbance 405 nm | 15-1 Absorbance 405 nm |
|---|---|---|---|
| 1073 | $10^5$ | 1.05 | 0.43 |
| | $10^3$ | 0.81 | 0.28 |
| | 10 | 0.49 | 0.20 |
| 15-1 | $10^5$ | 0.60 | 0.78 |
| | $10^3$ | 0.44 | 0.59 |
| | 10 | 0.41 | 0.33 |
| unrelated | $10^5$ | 0.31 | 0.18 |
| NMIg | $10^5$ | 0.29 | 0.17 |

These results show that binding was restricted to the specific Id determinants of the homologous mAb. It should be noted that neither anti-Id l073 nor anti-Id l5-l reacted with heterologous mAb, other than mAb l073 or mAb l5-l. The partial cross reactivity of mAb l5-l with anti-Id l073 and vice versa demonstrates that mAb l5-l and mAb l073 recognize a common epitope on the E. tenella sporozoite.

Cross reactivity was further evaluated by competitive inhibition of binding of AP-labeled anti-Id antibody to the homologous mAb by different unlabeled anti-Id antibodies in an ELISA test, see Example 2. In this assay 5 μg of mAb was absorbed on to the plastic surface of the well and reacted with l0 μg of AP-labeled anti-Id preincubated with different concentrations of various unlabeled anti-Id antibodies. There was l00% inhibition of binding of anti-Id 1073 to its homologous mAb by excess unlabeled anti-Id 1073. A partial inhibition, 43%, of binding of anti-Id 1073 to mAb 1073 was observed when mAb 15-1 was reacted with anti-Id 1073. The binding of anti-Id 1073 or that of anti-Id 15-1 to their homologous mAb was not affected by other anti-Id antibodies.

The IgG fraction of the anti-Id antisera was obtained by protein-A Sepharose affinity chromatography. The procedure is described in Example I.

Paratope-associated Id determinants were evaluated by the ability of anti-Id to inhibit the binding of mAb to sporozoite antigen immobilized on a plastic surface. In this assay a predetermined dilution of mAb, one which gives an absorbance reading of 0.7-0.9 in ELISA, was incubated with serial dilutions of anti-Id for 30 minutes at 4°C. The pre-incubated mixture was then allowed to react with sporozoite antigen, 10 μg. After washing and blocking the plates were incubated with AP-labeled goat anti-rabbit IgG and color developed with substrate as described in Example 2. The binding of mAb 1073 and mAb 15-1 to sporozoite antigen was specifically inhibited by homologous anti-Id. In addition, the binding of mAb 1073 by anti-Id 15-1 was partially inhibited and vice versa. The soluble sporozoite antigen, described in Example I, caused only partial, 51%, inhibition of the binding of anti-Id 1073 to its homologous mAb. Similarly there was a partial inhibition, 39%, of binding of the anti-Id 15-1 to mAb 15-1. Both results demonstrate that the Id determinants of mAb 1073 and mAb 15-1 were associated with the paratope.

The association of Id with the paratope of the mAb was confirmed by the ability of free sporozoite antigen to inhibit the binding of mAb to homologous anti-Id antibody. In this binding assay a predetermined dilution of anti-Id antibody was preincubated with various concentrations of solubilized sporozoite antigen and then the reaction mixture reacted with mAb coated on a plastic surface. These findings further confirmed that the Id was associated with the paratope of the mAb.

## EXAMPLE 4

### Production and Characterization of Anti-Id Antibodies in Chickens

Purified anti-E. tenella sporozoite monoclonal antibodies from Example 2 were used to produce anti-Id 1073 antibodies in chickens. Leghorn chickens, 6 to 8 weeks of age, were immunized by three weekly intramuscular injections of mAb 1073 in Freund's complete adjuvant. A total of I mg mAb in 0.5 ml was emulsified with 0.5 ml Freund's complete adjuvant to give I ml of a water-in-oil emulsion. The immunogen was given at multiple sites in the breast muscle. One week after the last injection the serum was collected, processed and frozen.

The chicken anti-Id 1073 was precipitated with 18% sodium sulfate, the precipitate was held in 18% sodium sulfate, resuspended in PBS and extensively dialyzed against PBS. Normal chicken serum was prepared in the same manner and used as a control. Chicken anti-Id 1073 was absorbed 16 times with an unrelated mAb IgG as in Example 3. This was insufficient to completely eliminate cross reactivity with other monoclonals. To obtain specificity the anti-Id 1073, 500 μg/ml, was admixed with unrelated mAb (500 μg/ml). The resultant immune complexes were removed by centrifugation. Specificity was also confirmed by an ELISA test, see Example 2. Dilutions of anti-Id 1073, 100 μl, were added to wells containing 5 μg of homologous mAb, heterologous mAb, or NMIg. The anti-Id antibody dilutions ranged from I:50 to I:3,200. The following results were obtained.

## Anti-Idiotypic Antibody 1073

| mAb | Anti-Id Reciprocal of Dilution | Optical Density $(X\ 10^{-3})$ |
|-----|--------------------------------|--------------------------------|
| 1073 | 50 | 1350 |
|  | 100 | 1130 |
|  | 200 | 800 |
|  | 400 | 550 |
| 15-1 | 50 | 240 |
|  | 100 | 150 |
|  | 200 | 110 |
|  | 400 | 100 |
| NMIg | 50 | 92 |
|  | 100 | 37 |
|  | 200 | 30 |
|  | 400 | 31 |

These results show that binding was restricted to the specific Id determinants of the homologous mAb 1073. In a competitive binding assay as described in Example 3, other mAb did not specifically inhibit the binding of anti-Id 1073 to mAb 1073. The binding of chicken anti-Id 1073 to mAb 1073 was not inhibited when soluble E. tenella sporozoite antigen was added to the ELISA competitive inhibition assay.

EXAMPLE 5

Immunization of Two-Day-Old Chickens Against Coccidiosis with Rabbit Anti-Idiotypic Antibodies

Broiler chicks were immunized intramuscularly with various rabbit anti-Id antibodies as detailed in Example 3. The anti-Id 1073 and anti-Id 15-1 were precipitated with aluminum gel (alum) according to the procedure of Sanchez et al . Infect. Immun. 30:728-733 (1980). The final percentage of aluminum gel per protein was 25 on a volume/volume basis. The first injection, at day 2, consisted of 50 μg of alum precipitated anti-Id or rabbit IgG (RIgG). The birds were boosted at day 15 with 50 μg of alum precipitated immunogen and at 22 days with 200 μg of the appropriate immunogen. One group of chickens was immunized on the same days with 10 μg of the soluble sporozoite immunogen described in Example I. All chickens were bled 2 weeks after the last immunization and challenged with an oral inoculation of $5 \times 10^3$ fully virulent E. tenella sporulated oocysts. Six days after challenge the chickens were killed and the severity of lesions in the ceca determined according to Johnson and Reid, Exp. Parasit 28:30-36 (1970). The lesions were scored on a defined scale of I to 4 where 4 is the most severe. Controls included rabbit IgG (RIgG) as described in Example 3 and sporozoite immunogen as described in Example I. The following results were obtained.

9

| Immunizing Agent | Number of Birds | Mean Cecal Lesion Score $\pm$ S.E |
|---|---|---|
| Anti-Id 1073 | 8 | 1.85 $\pm$ 0.13 |
| Anti-ID 15-1 | 8 | 1.50 $\pm$ 0.18 |
| RIgG | 4 | 3.50 $\pm$ 0.14 |
| Sporozoite Immunogen | 8 | 1.08 $\pm$ 0.16 |
| None | 8 | 3.40 $\pm$ 0.15 |

These results show that specific anti-Id produced in response to monoclonal antibody reactive with E. tenella sporozoite surface antigens can be used to immunize 2-day-old broilers against coccidiosis. Immunization with anti-Id 15-1 and anti-Id 1073 provides a high level of protection against the disease as indicated by the significant reduction in lesions developing in immune birds after challenge.

## EXAMPLE 6

### Immunization of Four-Day-Old Chickens Against Coccidiosis with Rabbit Anti-Idiotypic Antibodies

Broiler chicks were immunized intramuscularly with rabbit anti-Id antibodies as detailed in Example 3. The birds were immunized on days 4, 18 and 30 with 50 $\mu$g, 200 $\mu$g and 200 $\mu$g of the appropriate anti-Id antibody. Sporozoite immunogen and RIgG concentrations were equivalent to those in Example 5. All chickens were challenged two weeks after the last immunization with an oral inoculation of $5 \times 10^4$ fully virulent E. tenella sporulated oocysts. Six days after challenge the chickens were killed and the severity of lesions in the ceca determined according method of Johnson and Reid, supra. The controls were described in Example 5. The lesions were scored as detailed in Example 5. The following results were obtained.

| Immunizing Agent | Number of Birds | Mean Cecal Lesion Score $\pm$ S.E |
|---|---|---|
| Anti-Id 1073 | 10 | 1.50 $\pm$ 0.28 |
| Anti-ID 15-1 | 10 | 1.70 $\pm$ 0.24 |
| Sporozoite Immunogen | 10 | 1.40 $\pm$ 0.15 |
| RIgG | 10 | 3.40 $\pm$ 0.22 |
| None | 10 | 3.10 $\pm$ 0.16 |

These results show anti-Id 1073 and anti-Id 15-I can be used to immunize chickens against coccidiosis. The chickens showed a high level of immunity as indicated by a significant reduction of lesions following challenge with an infective dose of E. tenella oocysts.

EXAMPLE 7

Immunization of Three-Day-Old Chickens Against Coccidiosis with Chicken Anti-Idiotypic Antibodies

Broiler chicks were immunized intramuscularly with chicken anti-Id 1073 as detailed in Example 4. The birds were immunized on days 3, 10, and 17 with 50 µg anti-Id 1073 conjugated to alum, see Example 5. Sporozoite immunogen was administered as in Example 5 while normal chicken serum (NCS) was administered at the same level at the anti-Id antibody. All chickens were challenged two weeks after the final immunization with an oral inoculation of $1 \times 10^4$ fully virulent E. tenella sporulated oocysts. Six days after challenge the chickens were killed and the severity of the lesions in the ceca was determined according to the method of Johnson and Reid, supra. The lesions were scored as detailed in Example 5. Controls included normal chicken serum (NCS) prepared in Example 4 and sporozoite immunogen as described in Example I. The following results were obtained.

| Immunizing Agent | Number of Birds | Mean Cecal Lesions ± S.E. |
|---|---|---|
| Anti-Id 1073 | 8 | 2.10 ± 0.59 |
| NCS | 8 | 3.90 ± 0.09 |
| Sporozoite Ag | 8 | 1.60 ± 0.39 |
| None | 8 | 3.60 ± 0.26 |

These results show that anti-Id 1073 raised in chickens can be used to immunize chickens against coccidiosis. The chickens showed a high level of immunity as indicated by the reduction in severity of lesions following challenge with an infective dose of E . tenella oocysts.

EXAMPLE 8

Immunization of Three-Day-Old Chickens Against Coccidiosis with Chicken Anti-Idiotypic Antibodies

Broiler chicks were immunized intramuscularly with chicken anti-Id 1073 as detailed in Example 4. The birds were immunized on days 3, 10 and 17 with 50 µg anti-Id 1073. Sporozoite immunogen was administered as in Example 5 and the NCS as in Example 7. All chickens were challenged one week after the final immunization with an oral inoculation of $1 \times 10^4$ virulent E . tenella sporulated oocysts. Six days after challenge the chickens were killed and the severity of the lesions in the ceca determined according to the method of Johnson and Reid, supra. The lesions were scored as detailed in Example 5. Controls are described in Example 7. The following results were obtained.

| Immunizing Agent | Number of Birds | Mean Cecal Lesions ± S.E. |
|---|---|---|
| Anti-Id 1073 | 8 | 0.70 ± 0.13 |
| NCS | 8 | 1.83 ± 0.25 |
| Sporozoite Im | 8 | 1.00 ± 0.18 |
| None | 8 | 1.25 ± 0.19 |

These results show that anti-Id 1073 raised in chickens can be used to immunize chickens against coccidiosis. The chickens showed a high level of immunity as indicated by the general absence of lesions following challenge with an infective dose of E . tenella oocysts.


EXAMPLE 9

Immunization of Three-Day-Old Chickens Against Coccidiosis with Chicken Anti-Idiotypic Antibodies

Broiler chicks were immunized intramuscularly with chicken anti-Id 1073 as described in Example 4. The birds were immunized on days 3, 10 and 17 with 100 μg anti-Id 1073 conjugated to alum as in Example 5. Sporozoite immunogen was administered as in Example 5 and the NCS as in Example 7. All chickers were challenged one week after the final immunization with an oral inoculation of $3.5 \times 10^3$ fully virulent E. tenella sporulated oocysts. Six days after challenge the chickens were killed and the severity of the lesions in the ceca determined according to the method of Johnson and Reid, supra. The lesions were scored as detailed in Example 5. Controls are described in Example 7. The following results were obtained.

| Immunizing Agent | Number of Birds | Mean Cecal Lesions ± S.E. |
|---|---|---|
| Anti-Id 1073 | 8 | 1.56 ± 0.10 |
| NCS | 8 | 2.35 ± 0.20 |
| Sporozoite Im | 8 | 1.00 ± 0.22 |
| None | 8 | 2.50 ± 0.17 |

These results show that anti-Id 1073 raised in chickens can be used to immunize chickens against coccidiosis of a wide dosage range. The anti-Id antibody provides a high level of protection against the disease as indicated by the reduction in the severity of lesions in immune birds after a normally virulent infection.

## EXAMPLE 10

### Characterization of Anti-Anti-Id 1073 and 15-1 Antibodies

The antibody produced by chickens (Ab3, anti-anti-Id) immunized with xenogeneic anti-Id (Ab2) should be related to the Id hearing mAb (Ab1) against which the anti-Id was raised. This was determined by analyzing the ability of sera from anti-Id immunized chickens to bind to sporozoite antigen, described in Example 1. Sera was collected prior to oocyte challenge as described in Example 3 and prior to lesion assay Example 5 and 6. Carboxylated microspheres (Polysciences, Inc.), 3 $\mu$ in diameter, 12.5 mg/ml were coated with sporozoite antigen, 1.8 mg/ml in PBS by incubation on ice for 1 hour. Antigen coated microspheres, 15 $\mu$l were incubated with appropriate dilutions of immune chicken sera, normal control sera, and standard hyperimmune chicken sera raised against E. tenella sporozoites. After washing with PBS containing 1% BSA (PBS-BSA), the microspheres were incubated for 1 hour at 4°C with 100 $\mu$l of a dilution of fluorescein labeled rabbit anti-chicken IgG, F(ab)$_2$ (Cappel Laboratories). The microspheres were washed with PBS-BSA, resuspended in 0.5 ml of PBS, 20,000 microspheres, and fluorescense measured by a FACS IV flow cytometer (Becton Dickinson). The following results were obtained.

Pre-oocyte Challenge

| Immunizing Agent | Number of Birds | Relative Intensity of Fluorescence (% Above Normal Serum) Mean ± S.E. |
|---|---|---|
| Anti-Id 1073 | 8 | $19.0 \pm 3.5$ |
| Anti-Id 15-1 | 8 | $20.5 \pm 3.5$ |
| NRIgG | 4 | $2.0 \pm 0.9$ |
| Sporozoite Im | 5 | $19.5 \pm 4$ |

Post-oocyte Challenge

| Immunizing Agent | Number of Birds | Relative Intensity of Fluorescence (% Above Normal (Serum) Mean ± S.E. |
|---|---|---|
| Anti-Id 1073 | 8 | $49.3 \pm 7$ |
| Anti-Id 15-1 | 8 | $58.0 \pm 10$ |
| NRIgG | 4 | $3.0 \pm 1.3$ |
| Sporozoite Im | 5 | $45.0 \pm 17$ |

These results show that anti-Id 1073 and anti-Id 15-l can immunize chickens to produce moderate levels of circulating anti-anti-Id antibody. Challenge with viable oocytes stimulates a significant increase in antibody levels.

Claims

1. A process for preparing hybridoma cells which produce IgG monoclonal antibody capable of binding to the surface of intact sporozoites and passively protecting chickens from coccidiosis comprising immunizing mice with purified Eimeria tenella sporozoites, fusing resulting antibody-producing spleen cells with mouse myeloma cells and selecting hybridoma cells.

2. A method according to Claim 1 wherein the antibody-producing spleen cells are obtained from immunized Balb/c mice.

3. A method according to Claim 1 wherein the myeloma cells are non-immunoglobulin producing mouse SP-2/0 cells.

4. A hybridoma according to Claim 1 designated as 1073.10 and having the ATCC number HB9017.

5. A hybridoma according to Claim 1 designated as 15-1 and having the ATCC number HB9016.

6. A monoclonal antibody which immunologically binds to a non-peptide surface component of E. tenella and having the subclass $IgG_3$ and designated mAb 1073.

7. A monoclonal antibody which immunologically binds to an E. tenella sporozoite surface polypeptide with a molecular weight of 82KD, having the subclass $IgG_2$ and designated mAb 15-1.

8. A process for preparing anti-idiotypic antibody in which a member of a mammalian or avian species is immunized with the monoclonal antibody of either Claim 6 or Claim 7, isolating and purifying the anti-idiotypic antibodies which immunologically react only with the paratope of the monoclonal antibody specific for the original antigen.

9. An anti-idiotypic antibody according to Claim 8 designated anti-Id 1073.

10. An anti-idiotypic antibody according to Claim 8 designated anti-Id 15-1.

11. An anti-idiotypic antibody raised by immunizing a mammalian or avian species with an anti-Eimeria sporozoite monoclonal antibody, isolating and purifying the anti-idiotypic antibodies which immunologically react only with the paratope of the monoclonal antibody specific for the Eimeria antigen.

12. A composition comprising the antibodies of any one of Claims 6, 7, 9, 10 or 11 and a physiologically acceptable medium.

13. The use of a monoclonal antibody as claimed in either Claim 6 or 7 for the manufacture of a medicament useful for passively protecting chickens against coccidiosis.

14. The use of the anti-idiotypic antibody as claimed in any one of Claims 9, 10 or 11 for the preparation of a medicament useful for immunizing chickens against coccidiosis.